# EUROPÄISCHE PATENTSCHRIFT

(11) **EP 2 763 741 B1**
(45) Veröffentlichungstag und Bekanntmachung des Hinweises auf die Patenterteilung: **07.06.2017**
(21) Anmeldenummer: 12766659.2
(22) Anmeldetag: 01.10.2012
(51) Int. Cl.: A61M 39/10

(54) **SCHNELLKUPPLUNGSVORRICHTUNG**
QUICK RELEASE DEVICE
DISPOSITIF DE CONNEXION RAPIDE

(30) Priorität: 05.10.2011 DE 102011084027
(43) Veröffentlichungstag der Anmeldung: 13.08.2014
(73) Patentinhaber: Maquet Cardiopulmonary AG, 76437 Rastatt (DE)
(72) Erfinder: HAAG, Ulrich, 72406 Bisingen (DE); ENGELHARDT, Ralf, 72411 Bodelshausen (DE)
(74) Vertreter: Kohler Schmid Möbus Patentanwälte
(86) Internationale Anmeldenummer: PCT/EP2012/069329
(87) Internationale Veröffentlichungsnummer: WO 2013/050319

(56) Entgegenhaltungen:
- WO-A2-2008/058134
- BE-A1- 893 623
- US-A- 5 693 025
- US-A1- 2005 082 828
- US-A1- 2007 249 197

## Beschreibung

Die Erfindung betrifft eine Schnellkupplungsvorrichtung für Fluid führende Schlauch- oder Rohrleitungen, insbesondere von extrakorporalen Blutkreisläufen, mit den Merkmalen des Oberbegriffs des Anspruchs 1.

Eine solche Schnellkupplungsvorrichtung ist aus der US 2007/249 197 A1 bereits bekannt. Allerdings weist diese Kupplungsvorrichtung keine Möglichkeit der Entlüftung auf und ist daher für extrakorporale Blutkreisläufe nur bedingt einsetzbar.

Die US 5,593,025 A beschreibt einen medizinischen Adapter mit einem weiblichen und männlichen Kupplungsstück, wobei am männlichen Kupplungsstück ein Anschlussrohrstück für einen Katheter angeordnet ist. Das Rohrstück weist keine verschließbare Öffnung auf. Eine Entlüftung des Adapters ist hier ebenfalls nicht möglich.

Aus der WO 2008/058134 A1 ist eine Gefäßzugangsvorrichtung mit einem Scheidewandventil bekannt. Mit diesem Ventil lässt sich Gas aus einem extrakorporalen Kreislauf entfernen. Es ist jedoch nicht ersichtlich, wie dieses Ventil an einer Schnellkupplungsvorrichtung angeordnet werden kann.

Zur Lösung der beschriebenen Probleme schlägt die vorliegende Erfindung eine Schnellkupplungsvorrichtung mit den Merkmalen des Anspruchs 1 vor.

Mit der erfindungsgemäßen Schnellkupplungsvorrichtung ist ein rascher und auch für Blut schonender Austausch von Komponenten im Fluidkreislauf möglich. Die laminare Fluidströmung wird aufrechterhalten. Im Kupplungsbereich entstehen keine Spalte oder Stufen, die zu Flüssigkeitsschädigungen führen könnten.

Dabei kann die Dichtung am männlichen Kupplungsstück zweckmäßigerweise aus einem elastisch nachgiebigen Material gefertigt sein. Dieses Material ist besonders gut in der Lage, den entsprechend geformten Hohlraum des weiblichen Kupplungsstücks axial und radial nach außen spielfrei auszufüllen.

Um das Entweichen jedweden Fluids zu vermeiden, ist es außerdem vorteilhaft, zwischen dem männlichen und weiblichen Kupplungsstück zusätzlich eine O-Ring-Dichtung vorzusehen.

Insbesondere für den Einsatz der Schnellkupplungsvorrichtung in extrakorporalen Blutkreisläufen weisen die Kupplungsstücke einen Bereich mit einem gegenüber den Schlauchanschlussbereichen vergrößerten Innendurchmesser auf, wobei in diesem Bereich eine verschließbare Entlüftungsöffnung vorgesehen ist. Im Bereich vergrößerten Innendurchmessers können sich ggf. im Blut vorhandene Gasblasen ansammeln, die über die Entlüftungsöffnung nach außen geleitet werden können. Im Blutkreislauf sind auch kleinste Gasblasen zu vermeiden, da ansonsten Embolien auftreten können. Nicht alle Komponenten im Blutkreislauf verfügen über eigene Entlüftungsmöglichkeiten. Die notwendige Entlüftung kann nun über die Schnellkupplung erfolgen.

Die Entlüftungsöffnung ist verschließbar, sodass nach der erfolgten Entlüftung sichergestellt werden kann, dass keine Flüssigkeit durch die Entlüftungsöffnung austritt.

Der Bereich vergrößerten Innendurchmessers kann nicht rotationssymmetrisch zur Längsachse der Kupplungsstücke sein und vorzugsweise von einem im oberen Bereich der Kupplungsstücke angeordneten Längskanal gebildet sein. Bei dieser Ausgestaltung können sich die Gasblasen bei geringen Strömungsgeschwindigkeiten im Bereich erweiterten Durchmessers ansammeln und vollständig über die Entlüftungsöffnung abgeleitet werden.

Insbesondere bei dieser nicht rotationssymmetrischen Ausgestaltung des Bereichs erweiterten Durchmessers ist es zweckmäßig, wenn die beiden Kupplungsstücke verdrehsicher miteinander verbindbar sind. Der Gassammelbereich beider Kupplungsstücke kann dadurch sicher in der notwendigen fluchtenden Stellung gehalten werden.

Zur Vermeidung von Turbulenzen in der Fluidströmung ist es außerdem von Vorteil, wenn der Kanal an seinen Enden einen allmählichen Übergang zu den Schlauchanschlussbereichen aufweist. Auch die Entlüftungsöffnung lässt sich im Bereich einer der Kanalenden, vorzugsweise demjenigen in Flussrichtung vorne liegenden Ende anordnen. Die angesammelten Gase werden dann durch die vorhandene Strömung zur Entlüftungsöffnung geleitet und können so schneller entfernt werden.

Die Erfindung betrifft außerdem eine Entlüftungsvorrichtung für eine Schnellkupplungsvorrichtung für Schlauch- oder Rohrleitungen nach einem der Ansprüche 1 bis 7 mit einem weiblichen und einem männlichen Kupplungsstück, die einen Bereich mit einem gegenüber den Schlauchanschlussbereichen vergrößerten Innendurchmesser aufweisen, in dem eine Entlüftungsöffnung vorgesehen ist, die dadurch gekennzeichnet ist, dass sie lösbar mit der Entlüftungsöffnung der Schnellkupplungsvorrichtung verbunden ist und einen Hohlraum aufweist, in dem ein poröses Material oder eine semipermeable Membran angeordnet ist.

Diese Entlüftungsvorrichtung erlaubt eine selbsttätige Entlüftung der Leitungen oder Fluidbehandlungskomponenten. Ist diese Entlüftungsvorrichtung an einer Schnellkupplungsvorrichtung vorgesehen, so findet die Entlüftung automatisch nach Schließen der Kupplung statt. Die Entlüftungsvorrichtung kann aber auch, insbesondere über eine Luer-Lock-Verbindung, mit jeder Entlüftungsöffnung einer Fluidleitung oder einer Fluid-Behandlungskomponente verbunden werden. Selbstverständlich kann die erfindungsgemäße Entlüftungsvorrichtung auch manuell betätigt werden.

Das poröse Material oder die poröse Membran können aus einem hydrophoben Material, insbesondere aus Polyethylen, Polypropylen oder Teflon gefertigt sein. Ein solches Material oder eine solche Membran lässt Gase durch, hydrophile Flüssigkeiten wie Blut werden jedoch durch die Oberflächenspannung gesperrt. Kompakte poröse Materialien können beispielsweise durch Pulversinterung aus den genannten Stoffen hergestellt werden. Bei Einsatz von Membranen können diese bei Bedarf mittels eines Stützkörpers verstärkt werden. Wenn ein Gemisch aus hydrophiler Flüssigkeit und Gas auf eine solche poröse Oberfläche trifft, kann das Gas durch diese entweichen, während die Flüssigkeit die Poren nicht benetzen kann und daher nicht nach außen dringen kann.
Bei Verwendung in Blutkreisläufen ist somit sichergestellt, dass kein Blut beim Entlüftungsvorgang austritt, was zu Infektionen des Bluts führen könnte.

Bei einer vorteilhaften Ausgestaltung kann die Entlüftungseinrichtung einen Zugang zur Zugabe einer Flüssigkeit von außen in die Schlauch-oder Rohrleitung und/oder zur Probenentnahme aufweisen. Damit ist es möglich, Medikamente in den Blutkreislauf zu geben. Außerdem können Proben des Bluts entnommen werden, ohne dafür den Patienten kanülieren zu müssen.

Der Zugang kann dabei durch ein am Ende des Hohlraums angeordnetes Ventil gebildet werden, das selbsttätig schließend ausgebildet und insbesondere ein Schlitzventil sein kann. Durch den Schlitz des Ventils kann eine Spritze eingeführt und durch diese Medikamente verabreicht oder eine Probe entnommen werden. Nach Herausziehen der Spritze schließt sich das Ventil wieder selbsttätig.

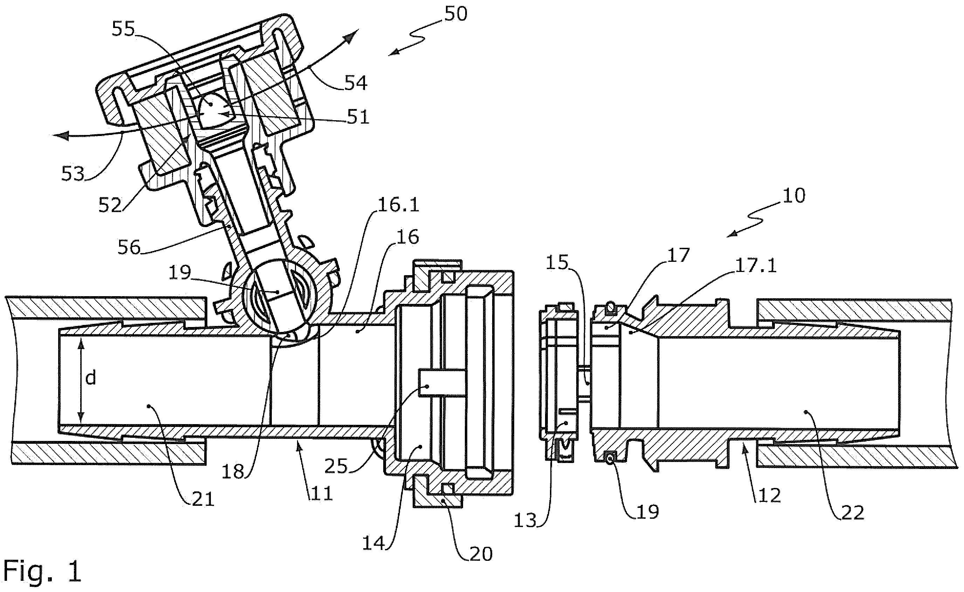

Im Folgenden wird ein bevorzugtes Ausführungsbeispiel einer erfindungsgemäßen Schnellkupplungsvorrichtung mit einer erfindungsgemäßen Entlüftungsvorrichtung anhand der Zeichnung näher beschrieben.

Die einzige Figur zeigt eine Schnittansicht einer Schnellkupplungsvorrichtung 10 für Schlauchleitungen (hier nicht dargestellt) sowie eine Entlüftungsvorrichtung 50.

Die Schnellkuppeleinrichtung weist ein weibliches Kupplungsstück 11 und ein männliches Kupplungsstück 12 auf. Das männliche Kupplungsstück 12 weist an seinem freien Ende eine Dichtung 13 auf, die in einen entsprechend geformten Hohlraum 14 im weiblichen Kupplungsstück 11 formschlüssig einführbar ist, wodurch die beiden Kupplungsstücke in radialer und in axialer Richtung spiel- und spaltfrei miteinander verbindbar sind. Die Dichtung 13 ist dabei an einem Vorsprung 15 befestigt, der in eine Tasche 25 des weiblichen Kupplungsstücks 11 einführbar ist und dadurch eine Verdrehsicherung der beiden Kupplungsstücke 11, 12 bildet.

Beide Kupplungsstücke 11, 12 weisen gegenseitig fluchtende Bereiche 16, 17 mit einem gegenüber den Schlauchanschlussbereichen 21, 22, die einen Innendurchmesser d haben, erweiterten Innendurchmesser auf, wobei sich diese Bereiche 16, 17 oben an den Kupplungsstücken 11, 12 befinden und gemeinsam einen Kanal bilden, in dem sich in der durch die Kupplungsstücke strömende Gase sammeln können. Die Enden 16.1 und 17.1 der Bereiche 16, 17 gehen jeweils allmählich in die Abschnitte 21, 22 über. Am Ende 16.1 des Bereichs 16 ist eine Entlüftungsöffnung 18 vorgesehen, die durch einen Hahn 19 verschließbar ist.

Werden die beiden Kupplungsstücke 11, 12 ineinander gefügt, so sorgt eine zusätzliche O-Ring-Dichtung (19) am männlichen Kopplungsstück 12 für eine fluiddichte Verbindung. Über einen Verriegelungsmechanismus 20 werden die beiden Kupplungsstücke gegen ein unbeabsichtigtes Lösen gesichert. Danach kann der Fluiddurchtritt durch die Schnellkupplungsvorrichtung freigegeben werden. Die im Fluid vorhandenen Gase setzen sich in den Bereichen 16, 17 ab und können über die Entlüftungsöffnung 18 bei geöffnetem Hahn 19 in die Entlüftungsvorrichtung 50 strömen.

In der Entlüftungsvorrichtung 50 ist ein Hohlraum 51 vorgesehen, dessen Wandung zumindest abschnittsweise von einer semipermeablen Membran 52 gebildet wird. Die passierenden Gase können seitlich in Richtung der Pfeile 53, 54 entweichen. An seinem oberen Ende ist der Hohlraum 51 durch ein Schlitzventil 55 verschlossen. Durch dieses Ventil 55 kann eine Spritze (nicht gezeigt) von außen bis in den Bereich des Hahnaustrittsstutzens 56, an der die Entlüftungsvorrichtung 50 mit einer Luer-Lock-Verbindung befestigt ist, eingeführt werden und damit Stoffe dem Fluid zugegeben oder Proben aus diesem entnommen werden.

## Patentansprüche

1. Schnellkupplungsvorrichtung für Fluid führende Schlauch- oder Rohrleitungen, insbesondere von extrakorporalen Blutkreisläufen, mit einem männlichen und einem weiblichen Kupplungsstück (11, 12), die lösbar und fluiddicht miteinander verbindbar sind, wobei am freien Ende des männlichen Kupplungsstücks (12) eine formschlüssig und in radialer und axialer Richtung spielfrei in eine entsprechend geformte Ausnehmung (14) des weiblichen Kupplungsstück (11) einführbare Dichtung (13) angeordnet ist, die nach dem Verbinden der Kupplungsstücke (11, 12) einen spalt- und stufenfreien Übergang zwischen den Fluid führenden Hohlräumen der beiden Kupplungsstücke (11, 12) ergibt, **dadurch gekennzeichnet, dass** die Kupplungsstücke (11, 12) einen Bereich (16, 17) mit einem gegenüber den Schlauchanschlussbereichen vergrößerten Innendurchmesser aufweisen und in diesem Bereich (16, 17) eine verschließbare Entlüftungsöffnung (18) vorgesehen ist.

2. Schnellkupplungsvorrichtung nach Anspruch 1, **dadurch gekennzeichnet, dass** die Dichtung (13) am männlichen Kupplungsstück (12) aus einem elastisch nachgiebigen Material gefertigt ist.

3. Schnellkupplungsvorrichtung nach Anspruch 1 oder 2, **dadurch gekennzeichnet, dass** zwischen dem männlichen und weiblichen Kupplungsstück (11, 12) zusätzlich eine O-Ring-Dichtung (19) vorgesehen ist.

4. Schnellkupplungsvorrichtung nach einem der Ansprüche 1 bis 3, **dadurch gekennzeichnet, dass** der Bereich (16, 17) vergrößerten Innendurchmessers nicht rotationssymmetrisch zur Längsachse der Kupplungsstücke (11, 12) ist.

5. Schnellkupplungsvorrichtung nach einem der Ansprüche 1 bis 4, **dadurch gekennzeichnet, dass** die beiden Kupplungsstücke (11, 12) verdrehsicher miteinander verbindbar sind.

6. Schnellkupplungsvorrichtung nach Anspruch 4 oder 5, **dadurch gekennzeichnet, dass** der Bereich (16, 17) vergrößerten Innendurchmessers von einem im oberen Bereich der Kupplungsstücke(11, 12) angeordneten Längskanal gebildet ist.

7. Schnellkupplungsvorrichtung nach Anspruch 6, **dadurch gekennzeichnet, dass** der Kanal an seinen Enden (16.1, 17.1) einen allmählichen Übergang zu den Schlauchanschlussbereichen aufweist.

8. Entlüftungsvorrichtung und Schnellkupplungsvorrichtung (10) für Schlauch- oder Rohrleitungen nach einem der Ansprüche 1 bis 7 **dadurch gekennzeichnet, dass** die Entlüftungsvorrichtung lösbar mit der Entlüftungsöffnung (18) der Schnellkupplungsvorrichtung (10) verbunden ist und einen Hohlraum (51) aufweist, in dem ein poröses Material oder eine semipermeable Membran (52) angeordnet ist.

9. Entlüftungsvorrichtung und Schnellkupplungsvorrichtung nach Anspruch 8, **dadurch gekennzeichnet, dass** das poröse Material oder die poröse Membran (52) aus einem hydrophoben Material, insbesondere aus Polyethylen, Polypropylen oder Teflon gefertigt ist.

10. Entlüftungsvorrichtung und Schnellkupplungsvorrichtung nach Anspruch 8 oder 9, **dadurch gekennzeichnet, dass** sie einen Zugang zur Zugabe einer Flüssigkeit von außen in die Schlauch- oder Rohrleitung und/oder zur Probenentnahme aufweist.

11. Entlüftungsvorrichtung und Schnellkupplungsvorrichtung nach Anspruch 10, **dadurch gekennzeichnet, dass** der Zugang durch ein am Ende des Hohlraums (51) angeordnetes Ventil (55) gebildet wird, das selbsttätig schließend ausgebildet und insbesondere ein Schlitzventil ist.

## Claims

1. A quick release device for fluid-conduction hose- or pipelines, in particular of extracorporeal blood circulations, with a male and a female release piece (11, 12) which can be connected to one another in a separable and fluid-tight manner, wherein a seal (13)which can be positively introduced and without play in the radial and axial direction into a correspondingly shaped recess (14) of the female coupling piece (11) is arranged on the free end of the male coupling piece (12),which seal results, after the connecting of the release pieces (11, 12) in a transition free of slots and graduations between the fluid-conducting hollow spaces of the two release pieces (11, 12), **characterized in that** the release pieces (11, 12) comprise an area (16, 17) with an inside diameter enlarged opposite the hose connection areas and that a closable ventilation opening (18) is provided in this area (16, 17).

2. The quick release device according to Claim 1, **characterized in that** the seal (13) on the male release piece (12) is manufactured from an elastically flexible material.

3. The quick release device according to Claim 1 or 2, **characterized in that** an O-ring seal (19) is additionally provided between the male and the female release piece (11, 12).

4. The quick release device according to one of Claims 1 to 3, **characterized in that** the area (16, 17) with an enlarged inside diameter is not rotationally symmetrical with the longitudinal axis of the release pieces (11, 12).

5. The quick release device according to one Claims 1 to 4, **characterized in that** the two coupling pieces (11, 12) can be connected to one another in a non-rotating manner.

6. The quick release device according to Claim 4 or 5, **characterized in that** the area (16, 17) with an enlarged inside diameter is formed by a longitudinal conduit arranged in the upper area of the coupling pieces (11, 12).

7. The quick release device according to Claim 6, **characterized in that** the conduit has a gradual transition on its ends (16.1, 17.1) to the hose connection areas.

8. The ventilation device and quick release device (10) for hose- or pipelines according to one of Claims 1 to 7, **characterized in that** the ventilation device is detachably connected to the ventilation opening (18) of the quick release device (10) and comprises a hollow space (51) in which a porous material or a semipermeable membrane (52) is arranged.

9. The ventilation device and quick release device according to Claim 8, **characterized in that** the porous material or the porous membrane (52) is manufactured from a hydrophobic material, in particular of polyethylene, polypropylene or Teflon.

10. The ventilation device and quick release device according to Claim 8 or 9, **characterized in that** it comprises an access for the adding of a liquid from the outside into the hose- or pipeline and/or for the taking of samples.

11. The ventilation device and quick release device according to Claim 10, **characterized in that** the access is formed by a valve (55) arranged on the end of the hollow space (51) and which is constructed to automatically close and is in particular a slot valve.

## Revendications

1. Dispositif de connexion rapide pour des conduites flexibles ou tubulaires acheminant du fluide, en particulier de circuits de sang extracorporels, comprenant des pièces de connexion (11, 12) mâle et femelle, qui peuvent être raccordées de manière amovible et de manière étanche aux fluides, un joint d'étanchéité (13) pouvant être introduit, par complémentarité de forme et dans une direction radiale et axiale, sans jeu, dans un évidement (14), formé de manière correspondante, de la pièce de connexion (11) femelle étant disposé au niveau de l'extrémité libre de la pièce de connexion (12) mâle, lequel joint d'étanchéité donne lieu, après le raccordement des pièces de connexion (11, 12), à une transition, dépourvue d'interstice et de palier, entre les cavités, acheminant du fluide, des deux pièces de connexion (11, 12), **caractérisé en ce que** les pièces de connexion (11, 12) présentent une zone (16, 17) présentant un diamètre intérieur agrandi par rapport aux zones de branchement de tuyaux flexibles, et **en ce qu'**une ouverture d'évacuation d'air (18) pouvant être fermée est prévue dans ladite zone (16, 17).

2. Dispositif de connexion rapide selon la revendication 1, **caractérisé en ce que** le joint d'étanchéité (13) est confectionné, au niveau de la pièce de connexion (12) mâle, à partir d'un matériau souple élastiquement.

3. Dispositif de connexion rapide selon la revendication 1 ou 2, **caractérisé en ce qu'**en supplément un joint torique (19) est prévu entre les pièces de connexion (11, 12) mâle et femelle.

4. Dispositif de connexion rapide selon l'une quelconque des revendications 1 à 3, **caractérisé en ce que** la zone (16, 17) à diamètre intérieur agrandi n'est pas symétrique en rotation par rapport à l'axe longitudinal des pièces de connexion (11, 12).

5. Dispositif de connexion rapide selon l'une quelconque des revendications 1 à 4, **caractérisé en ce que** les deux pièces de connexion (11, 12) peuvent être raccordées l'une à l'autre de manière bloquée en rotation.

6. Dispositif de connexion rapide selon la revendication 4 ou 5, **caractérisé en ce que** la zone (16, 17) à diamètre intérieur agrandi est formée par un canal longitudinal disposé dans la zone supérieure des pièces de connexion (11, 12).

7. Dispositif de connexion rapide selon la revendication 6, **caractérisé en ce que** le canal présente au niveau de ses extrémités (16.1, 17.1) une transition progressive en direction des zones de branchement de tuyaux flexibles.

8. Dispositif d'évacuation d'air et dispositif de connexion rapide (10) pour des conduites en tuyau flexible et des conduites tubulaires selon l'une quelconque des revendications 1 à 7,
**caractérisés en ce**
**que** le dispositif d'évacuation d'air est raccordé de manière amovible à l'ouverture d'évacuation d'air (18) du dispositif de connexion rapide (10) et présente une cavité (51), dans laquelle un matériau poreux ou une membrane (52) semi-perméable est disposé(e).

9. Dispositif d'évacuation d'air et dispositif de connexion rapide selon la revendication 8, **caractérisés en ce que** le matériau poreux ou la membrane poreuse (52) est confectionné(e) à partir d'un matériau hydrophobe, en particulier à partir de polyéthylène, de polypropylène ou de Téflon.

10. Dispositif d'évacuation d'air et dispositif de connexion rapide selon la revendication 8 ou 9, **caractérisés en ce qu'**ils présentent un accès servant à déverser un liquide depuis l'extérieur dans la conduite en tuyau flexible ou tubulaire et/ou servant à retirer des échantillons.

11. Dispositif d'évacuation d'air et dispositif de connexion rapide selon la revendication 10, **caractérisés en ce que** l'accès est formé par une soupape (55) disposée au niveau de l'extrémité de la cavité (51), laquelle est réalisée de manière à être à fermeture automatique et est en particulier une soupape à fente.
